(19) Europäisches Patentamt
European Patent Office
Office européen des brevets

(11) EP 1 586 638 A1

(12) **EUROPEAN PATENT APPLICATION**

(43) Date of publication:
**19.10.2005 Bulletin 2005/42**

(51) Int Cl.[7]: **C12N 15/06**, C12N 5/20, C07K 16/44

(21) Application number: **05105334.6**

(22) Date of filing: **17.02.1999**

(84) Designated Contracting States:
**AT BE CH CY DE DK ES FI FR GB GR IE IT LI LU MC NL PT SE**

(30) Priority: **26.02.1998 JP 4518598**

(62) Document number(s) of the earlier application(s) in accordance with Art. 76 EPC:
**99905209.5 / 1 057 890**

(71) Applicant: **Japan Envirochemicals, Ltd.**
**Osaka-shi, Osaka (JP)**

(72) Inventors:
- **Toyoda, Yukio**
  **Amagasaki-shi, Hyogo 661-0033 (JP)**
- **Fujita, Masanori**
  **Suita-shi, Osaka 565-086 (JP)**
- **Goda, Yasuhiro**
  **Ibaraki-shi, Osaka 567-0855 (JP)**
- **Miyagawa, Ken-ichiro**
  **Toyono-gun, Osaka, 563-0103 (JP)**
- **Fujimoto, Shigeru**
  **Ikeda-shi, Osake 563-0032 (JP)**
- **Kobayashi, Ayako**
  **Nishinomiya-shi, Hyogo 663-8003 (JP)**
- **Fukuda, Katsuji**
  **Ikeda-shi, Osaka 563-0043 (JP)**

(74) Representative: **von Kreisler Selting Werner**
**Patentanwälte**
**P.O. Box 10 22 41**
**50462 Köln (DE)**

Remarks:
This application was filed on 16.06.2005 as a divisional application to the application mentioned under INID code 62.

(54) **Monoclonal antibody for immunologically analyzing or concentrating endocrine disruptor or its degradation product and utilization of the same**

(57) A hybridoma which produces a monoclonal antibody against an endocrine disruptor or its degradation product obtained by fusing spleen cells or lymphoid cells of an animal having been immunized with a complex of the endocrine disruptor or a compound similar thereto with a protein with myeloma cells; the monoclonal antibody produced thereby; and a method for immunologically detecting the endocrine disruptor or its degradation product and a method for immunologically concentrating the same each by using the above antibody.

EP 1 586 638 A1

## Description

Art Field Related

The present invention relates to a monoclonal antibody for immunologically analyzing or concentrating an endocrine disruptor or its degradation product and its use. More specifically, it relates to a hybridoma which produces the monoclonal antibody, the monoclonal antibody produced by the hybridoma, and a kit and a method for immunologically analyzing or concentrating an endocrine disruptor, its degradation product or a mixture thereof each by using the monoclonal antibody.

Background Art

Endocrine disruptors are also referred to as endocrine disrupting chemicals or environmental hormones. Recently, a social problem has been caused by environmental pollution due to them. Then, it is necessary to determine and analyze endocrine disruptors and their degradation products in an environment and to utilize the results for environmental preservation.

Various methods for determination and analysis of such endocrine disruptors and their degradation products have been known heretofore in the prior art. For example, for quantitative determination of endocrine disruptors and their degradation products in tap water, rivers, swamps, lakes or sewage, there are high-performance liquid chromatography (HPLC), gas chromatography (GC), gas chromatography-high resolution mass spectrometry (GC-HRMS) and high-performance liquid chromatography-high resolution mass spectrometry (HPLC-HRMS), and the like.

However, in these known methods, there are such problems that sensitivity of these methods are insufficient for that required for determination of endocrine disruptors which are present in trace amounts in specimens; determination requires high concentration of specimens, for example, by extraction with organic solvents; and the methods require very expensive apparatuses or devices, pretreatment and operation with great skill. In view of these problems, it is desired to develop a method for determination of endocrine disruptors which is highly sensitive and can be readily and quickly operated with high specificity at a low cost.

As means for solving the above problems, there are an enzyme-linked immunosorbent assay (hereinafter sometimes abbreviated to ELISA) and immunological concentration.

Assay systems and kits utilizing ELISA have been constructed for various environmental contaminants. For example, WO 94/12536 discloses ELISA for detecting petroleum fuel in an environment and a kit for such ELISA. The kit comprises an enzyme and an antibody, and the measurement can be completed very quickly, usually, within several hours. And, the operation is very simple in comparison with conventional HPLC, GC, GC-HRMS and HPLC-HRMS. Further, a very specific determination can be carried out by using an antibody, in particular, a monoclonal antibody. HPLC, GC, GC-HRMS and HPLC-HRMS require very expensive apparatuses and devices and their maintenance is also expensive, while ELISA has no such problem.

In addition, for accurate determination of a trace amount of an endocrine disruptor, its degradation product and a mixture thereof, high concentration of such a material is required. However, in conventional solvent extraction, solid phase extraction process etc., the degree of concentration cannot be raised higher than a certain level due to large amounts of contaminants. On the other hand, when concentration is carried out by utilizing an antibody, in particular, a monoclonal antibody, this problem does not caused because this concentration utilizes an antigen-antibody reaction.

Nevertheless, any method for immunological analysis method which can be used for detection and determination of endocrine disruptors and their degradation products, in particular, ELISA has not yet been established. Further, any immunological concentration method which can be used for selective concentration of an endocrine disruptor and its degradation product has not yet been established.

Objects of the Invention

One object of the present invention is to provide an immunological analysis method which can detect and determine endocrine disruptors and their degradation products.

Another object of the present invention is to provide an immunological method for selective concentration of endocrine disruptors and their degradation products.

These objects as well as other objects and advantages of the present invention will apparent to those skilled in the art from the following description with reference to the accompanying drawings.

Brief Explanation of Drawings

Fig. 1 illustrates chemical structures for haptens against respective compounds prepared in Example 1 (1), hereinafter.

Fig. 2 illustrates a calibration curve of alkylphenol ethoxylates (nonylphenol ethoxylate) prepared in Example 2, hereinafter.

Fig. 3 illustrates a calibration curve of plastic resin components (bisphenol A) prepared in Example 2, hereinafter.

Fig. 4 illustrates a calibration curve of alkylphenols (nonylphenol) prepared in Example 3, hereinafter.

Fig. 5 illustrates a calibration curve of chlorophenols (2,4,6-trichlorophenol) prepared in Example 3, hereinafter.

Fig. 6 illustrates a calibration curve of a plastic plasticizers (dibutyl phthalate) prepared in Example 3, hereinafter.

Summary of the Invention

The present inventors have studied to establish a method for immunological analysis of endocrine disruptors such as alkylphenol ethoxylates (hereinafter, sometimes, abbreviated to APE), alkylphenols (hereinafter, sometimes, abbreviated to AP), plastic resin components (hereinafter, sometimes, abbreviated to PRC), plastic plasticizers (hereinafter, sometimes, abbreviated to PP), chlorophenols (hereinafter, sometimes, abbreviated to CP) and their degradation products, in particular, a method for detection and determination thereof by ELISA, as well as a method for immunological concentration thereof, intensively. As a result, it has been found that a hybridoma which can produce an anti-endocrine disruptor-monoclonal antibody can be obtained by binding a compound similar to an endocrine disruptor to a carrier protein, immunizing an animal with the resultant complex material and isolating the hybridoma from its spleen. In addition, it has also been found that an endocrine disruptor and its degradation product can be determined with high sensitivity by using the above monoclonal antibody and the labeled endocrine disruptor and its degradation product. Further, it has been found that an endocrine disruptor, its degradation product and a mixture thereof can be highly concentrated by selectively adsorbing the above chemical to said monoclonal antibody by means of an antigen-antibody reaction and eluting the substance. On the basis of these findings, the present inventors have continued to study and have completed the present invention.

That is, according to the present invention, there is provided a hybridoma obtainable by fusing spleen cells or lymphocytes of an animal immunized by a complex of an endocrine disruptor or a compound similar thereto and a protein with myeloma cells, said hybridoma producing a monoclonal antibody against the endocrine disruptor or its degradation product.

The present invention also provides a monoclonal antibody against an endocrine disruptor or its degradation product which is produced by the hybridoma of the present invention.

Further, the present invention provides a kit for immunological analysis of an endocrine disruptor, its degradation product or a mixture thereof comprising as an essential constitutional component the monoclonal antibody of the present invention.

Furthermore, the present invention provides a method for immunological analysis of an endocrine disruptor, its degradation product or a mixture thereof in a specimen, in particular, ELISA which comprises reacting the specimen with the monoclonal antibody of the present invention supported on a carrier, and the endocrine disruptor, its degradation product or the mixture thereof labeled with a labeling agent, and then determining an activity of the labeling agent either supported on the carrier or not supported on the carrier.

The present invention further provides a kit for immunological concentration of an endocrine disruptor, its degradation product or a mixture thereof comprising as an essential constitutional component the monoclonal antibody of the present invention.

Moreover, the present invention provides a method for immunological concentration of an endocrine disruptor, its degradation product or a mixture thereof in a specimen which comprises subjecting the specimen to an antigon-antibody reaction with the monoclonal antibody of the present invention to bind the endocrine disruptor, its degradation product or a mixture thereof in the specimen to the monoclonal antibody.

The objective endocrine disruptors in the present invention includes alkylphenol ethoxylates, alkylphenols, plastic resin components, plastic plasticizers and chlorophenols. In addition, the objective endocrine disruptors of the present invention include various environmental contaminants, agrochemicals, heavy metals, synthetic estrogens, foods, food additives and the like. According to the present invention, these endocrine disruptors, their degradation products or a mixture thereof can be analyzed immunologically with high sensitivity and specificity, and can be highly concentrated.

Detailed Description of the Invention

The hybridoma of the present invention can be produced by using a complex of an endocrine disruptor or a compound similar thereto and a protein (carrier protein) according to a per se known method.

Examples of the endocrine disruptors to be used include:

Alkylphenol ethoxylates (APE) represented by the formula (1):

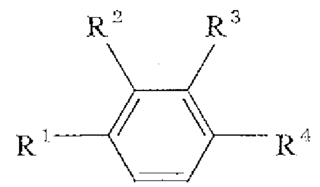

(1)

wherein $R^1$, $R^2$ and $R^3$ are the same or different and each is H or straight or branched (including sec-, tert-, iso-) alkyl having 1 to 20, preferably 1 to 12 carbon atoms; $R^4$ is $(OC_2H_4)_mOH$ or $(OC_2H_4)_mCOOH$; and m is the average number of ethylene oxide chain of 1 to 70, preferably 1 to 15 [e.g., NPE (nonylphenol ethoxylate, e.g., NP2EO (the average number of ethylene oxide chain = 2), NP5EO (the average number of ethylene oxide chain = 5), NP10EO (the average number of ethylene oxide chain = 10), NP10EC (the average number of ethylene oxide chain = 10, the terminal OH→ carboxylic acid), OPE (octylphenol ethoxylate), and the like];

Alkylphenols (AP) represented by the formula (2):

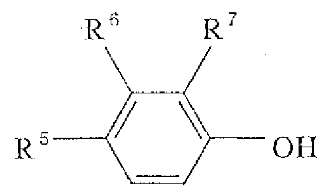

(2)

wherein $R^5$, $R^6$ and $R^7$ are the same or different and each is H or straight or branched (including sec-, tert-, iso-) alkyl group having 1 to 20, preferably 1 to 12 carbon atoms [e.g., DP (4-dodecylphenol), EP (4-ethylphenol), HP (heptylphenol), IPP (4-isopentylphenol), 2-OP (2-octylphenol), 4-NP (4-nonylphenol), 4-OP (4-octylphenol), 4-sBP (4-sec-butylphenol), 4-tBP (4-tert-butylphenol), 4-tPP (4-tert-pentylphenol), 4-tOP (4-tert-octylphenol), and the like];

Plastic resin components (PRC) represented by the formula (3) :

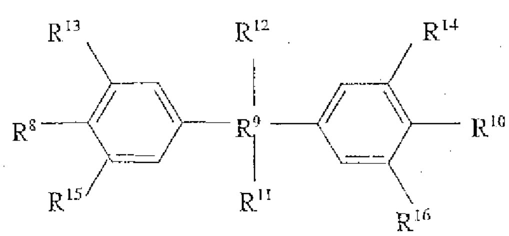

(3)

wherein $R^9$ is C, CO or $SO_2$; $R^8$ and $R^{10}$ are the same or different and each is H, OH, $NH_2$ or $O(CH_2)_2OH$; $R^{11}$ and $R^{12}$ are the same or different and each is absent, H, $CH_3$, $CH_2OH$ or $C_2H_4COOH$; and $R^{13}$, $R^{14}$, $R^{15}$ and $R^{16}$ are the same or different and each is H, OH, $CH_3$, Cl or Br [e.g., BPA (bisphenol A), 4,4'-EBP (4,4'-ethylidene bisphenol), BHPM (bis(p-hydorxyphenyl)methane), 2,2'-BHPP (2,2'-bis(4-hydroxyphenyl)-1-propanol), 2,2'-BMHPP (2,2'-bis(m-methyl-p-hydroxyphenyl)propane), 4,4'-BHPP (4,4'-bis(p-hydroxyphenyl)pentanoic acid), 4,4'-DDM (4,4'-diaminodiphenylmethane), 4,4'-DDE (4,4'-dihydroxydiphenyl ether), 4,4'-DOHDS (4,4'-dihydroxydiphenylsulfone), 4,4'-DClDS (4,4'-dichlorodiphenylsulfone), BHEDBrPS (bis[4-(2-hydroxyethoxy)-3,5-diburomophenyl]sulfone),

BHEPS (bis[4-(2-hydroxyethoxy)phenyl]sulfone], 4,4'-DDE (4,4'-dihydroxydiphenylether), p,p'-DBP (p,p'-dihydorxybenzophenone), HBP (4-hydroxybiphenyl), and the like] ;

Plastic plasticizers (PP) represented by the formula (4):

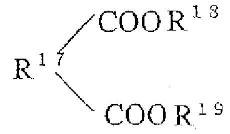

(4)

wherein $R^{17}$ is o-phenylene or tetramethylene; and $R^{18}$ and $R^{19}$ are the same or different and each is H, straight or branched (including sec-, tert-, iso-) alkyl having 1 to 20, preferably 1 to 12 carbon atoms, benzyl or cyclohexyl [e.g., BBP (butyl benzyl phthalate), DBP (dibutyl phthalate), DCHP (dicyclohexyl phthalate), DEP (diethyl phthalate), DEHP (di(2-ethylhexyl) phthalate), DEHA (diethyl hexyl adipate), DHP (dihexyl phthalate), DPP (di-n-pentyl phthalate), DPrP (dipropyl phthalate), and the like]; and

Chlorophenols (CP) represented by the formula (5):

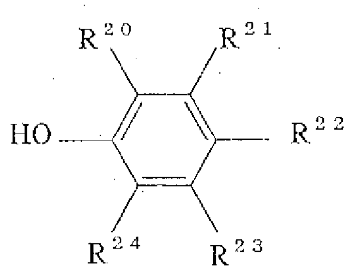

(5)

wherein $R^{20}$, $R^{21}$ $R^{22}$, $R^{23}$ and $R^{24}$ are the same or different and each is H or Cl [e.g., 2-CP (2-chlorophenol), 3-CP (3-chlorophenol), 4-CP (4-chlorophenol), 2,3-CP (2,3-dichlorophenol), 2,4-CP (2,4-dichlorophenol), 2,5-CP (2,5-dichlorophenol), 2,6-CP (2,6-dichlorophenol), 2,3,4-CP (2,3,4-trichlorophenol), 2,4,5-CP (2,4,5-trichlorophenol), 2,4,6-CP (2,4,6-trichlorophenol), 2,3,4,5-CP (2,3,4,5-tetrachlorophenol), 2,3,4,6-CP (2,3,4,6-tetrachlorophenol), PCP (pentachlorophenol), and the like]. These compounds can be appropriately selected according to a particular endocrine disruptor and a degradation products thereof to be analyzed or concentrated.

Further, the endocrine disruptors of the present invention include environmental contaminants (e.g. PCB, benzophenone, benzopyran, chlorobenzene, bromonaphthol, nitrotoluene, dioxine, chlorobenzofuran tributyltin, etc.), various agrochemicals, heavy metals (e.g., Cd, Hg, Pb, etc.), synthesized estrogens (e.g., centchroman, ethinyl estradiol, diethylstilbestrol, hexestrol, 2-hydroxyestradiol, tamoxifen, laroxyfen, etc.) foods, food additives [e.g., BHA (butyl hydroxy anisol), ecol, enterolactone, phytoestrogen, coumestrol, formononetin, daidzein, biotinin A, gestenin, etc.], and the like.

As the carrier protein, for example, there are bovine serum albumin (hereinafter abbreviated to BSA), ovalbumin (hereinafter abbreviated to OVA), keyhole limpet hemocyanin (hereinafter abbreviated to KLH), bovine thyroglobulin (hereinafter abbreviated to BTG), and the like.

The formation of the complex of the carrier protein and the endocrine disruptor or a compound similar thereto can be carried out by binding a compound represented by the formula (6):

$$A\text{-}R \qquad (6)$$

wherein R is COOH, $NH_2$ or SH; and A is a group which forms the endocrine disruptor or the compound similar thereto by removal of the group R, to the carrier protein according to a per se known method.

The compound represented by the formula (6) can also be synthesized chemically by introducing or forming a carboxyl group, amino group or sulfhydryl group in a suitable starting material according to a per se known method.

For example, the compound represented by the formula (6) wherein R is COOH and A is a group which forms a polyoxyethylene alkylphenyl ether can be produced by subjecting a polyoxyethylene alkylphenyl ether and succinic

anhydride to dehydration-condensation (formation of half ester) [Cancer Biochem. Biophys., 7, 175 (1984)].

The compound represented by the formula (6) wherein R is $NH_2$ and A is a group which forms a polyoxyethylene alkylphenyl ether can be produced by chlorinating the hydroxyl group of the polyoxyethylene alkylphenyl ether with thionyl chloride [J. Am. Chem. Soc., 60, 2540 (1938)], followed by treatment with ammonia (Organic Functional Group Preparations, Vol. 1, p. 382].

The compound represented by the formula (6) wherein R is SH and A is a group which forms a polyoxyethylene alkylphenyl ether can be produced by chlorinating hydroxyl group of the polyoxyethylene alkylphenyl ether with thionyl chloride [J. Am. Chem. Soc., 60, 2540 (1938)], followed by reaction with sodium hydrosulfide [J. Am. Chem. Soc., 72, 1843 (1950)].

For immunization of an animal, the complex obtained above was inoculated into the animal. Examples of the animal to be inoculated include goat, sheep, rabbit, rat, mouse, guinea pig, chicken and the like. In case that a monoclonal antibody against an endocrine disruptor (hereinafter sometimes abbreviated to MoAb) is desired, a mouse is particularly preferred.

The inoculation can be carried out according to a conventional method. For example, a mouse is inoculated with about 1 to 100 μg, preferably about 50 to 100 μg of the immunogen which is emulsified with equal amounts (0.1 ml) of physiological saline and Freund's complete adjuvant or RIBI adjuvant system™ per once at the back or abdomen subcutaneously or intraperitoneally. The mouse receives the inoculation 3 to 6 times at 2 to 3 week intervals.

Among the immunized animals, for example, mice, an individual having a high antibody titer is selected and, 3 to 5 days after the last immunization, the spleen or lymph node is collected. Then, the antibody producing cells contained therein are fused together with myeloma cells.

The fusion can be carried out by a known method and, for example, polyethylene glycol (hereinafter abbreviated to PEG) or Sendai virus can be used as a fusion promoting agent. PEG is preferred.

As myeloma cells, for example, NS-1, P3U1 or Sp2/O can be used and, in particular, P3U1 is preferred. For example, the preferred proportion of the spleen cells : myeloma cells is 1 : 1 to 10 : 1 and PEG having a molecular weight of about 1,000 to 6,000 is added thereto at a concentration of about 10 to 80%. The mixture is incubated at about 20 to 37°C, preferably, at about 30 to 37°C for about 3 to 10 minutes.

For screening of the desired hybridoma, various methods can be employed. For example, there is ELISA, wherein a supernatant of a hybridoma culture is added to a microplate on which OVA bound to a compound similar to an endocrine disruptor (hapten) has been adsorbed and then an anti-mouse immunoglobulin antibody labeled with horseradish peroxidase (hereinafter abbreviated to HRP) is added thereto to detect the antibody bound to the solid phase of the plate. A hybridoma having positive antibody activity is subjected to cloning immediately. Normally, this can be readily carried out by limiting dilution, or the like.

The antibody titer of a supernatant of cloned hybridoma is determined by the above method and a hybridoma which constantly produces an antibody having high titer is selected to obtain the desired monoclonal hybridoma.

Examples of the anti-endocrine disruptor-antibody producing hybridoma obtained by the above described method include hybridomas MOF3-139, AP-14, BP2-177, DF-34 and CP-8 as shown in the Examples.

The monoclonal antibodies against endocrine disruptors or their degradation products which are produced by these hybridomas are also included in the scope of the present invention.

The production of the monoclonal antibody by the hybridoma and the purification thereof can also be carried out by a per se known method. The monoclonal antibody thus obtained can serve as an antibody against not only endocrine disruptors or their degradation products, but also the compound of the formula (6).

The monoclonal antibody thus obtained can be prepared in the form of a solid phase antibody or an immunoadsorbent by a known method described in "Taisha", Vol. 8, 696 (1971) such as bromocyanate method, glutaraldehyde (hereinafter abbreviated to GLA) method, carbodiimide method, epoxy activation method, and the like. Further, as more preferred, simpler and easier methods, there are a method wherein the antibody is physically adsorbed on, for example, a 96-well microplate, tube, glass beads, polystyrene-latex or polystyrene particles, a method utilizing immunochromatography, and the like.

These can be combined with enzyme preparations for ELISA, buffers and the like to obtain kits for immunological analysis or for immunological concentration of endocrine disruptors, their degradation products or a mixture thereof. Such kits are also included in the scope of the present invention.

In the immunological analysis of the present invention, a so-called competitive assay is employed. That is, the quantitative determination can be carried out by quantitatively measuring competition between a specimen (e.g., water and soil samples containing an endocrine disruptor, it degradation product or a mixture thereof) and a known amount of a labeled endocrine disruptor, its degradation product or a mixture thereof for binding to the monoclonal antibody of the present invention. In such a competitive assay, a given amount of an antibody supported on a carrier is added to a specimen solution containing an unknown antigen, and further, a given amount of an antigen labeled with a labeling agent is added. The activity of the labeling agent supported by the carrier or the amount of the labeling agent which does not supported by the carrier is measured. Preferably, the addition of the antibody and that of the labeled

antigen are carried out almost at once.

In generally, the competitive assay can be carried out by a simpler and easier operation within a shorter period of time compared with sandwich method or the like.

Examples of the agent for labeling the antigen include radioisotopes (hereinafter abbreviated to RI), enzymes, enzyme substrates, fluorescent materials, biotin and the like. For binding the labeling agent to the antigen, maleimide method [J. Biochem, 79, 233 (1976)], active biotin method [J.. Am. Chem. Soc., 100, 3585 (1978)] and the like can be used.

For example, in case of an endocrine disruptor, a specific immunochemical determination by the competitive assay can be carried out by adding a solid phase, to which the antibody is bound physically or chemically by a know conventional means, to a sample to be tested, which contains an unknown amount of endocrine disruptors to allow to react. At the same time, a given amount of the antigen labeled with a labeling agent is added thereto to allow to react. Then, normally, the solid phase is washed thoroughly and the activity of the labeling agent bound to the solid phase is measured. When the labeling agent is RI, the measurement is carried out with a well counter or a liquid scintillation counter. When the labeling agent is an enzyme, its substrate is added, the mixture is allowed to stand and then the enzymatic activity is measured by colorimetry or fluorometry. When the labeling agent is a fluorescent material or a luminous material, it is measured according to a known method.

Since the monoclonal antibody having a high specificity is used, the immunological analysis of the present invention has such a very advantageous characteristic that, in case of APE, the specific determination of APE and their degradation products can be carried out without an error due to cross reaction with compounds other than APE and their degradation products, for example, compounds having a benzene ring such as toluene, phenol, benzene and aniline, or other endocrine disruptors.

Thus, in the present invention, by using the antibody having very high specificity for endocrine disruptors and their degradation products, influence of other chemical substances in a specimen can be eliminated and therefore the determination can be simply and readily carried out with high sensitivity and specificity. Therefore, the present invention is useful for not only academic analyses but also investigation of influence of endocrine disruptors and their degradation products on environmental preservation and the like.

Further, in the immunological concentration method of the present invention, the desired objective material can be concentrated at a high concentration such as several thousands to tens of thousands times with less immunological contaminants by passing a large amount of a specimen through an immunoadsorbent column, or mixing it with immunoadsorbent particles to bind the objective endocrine disruptor, its degradation product or a mixture thereof on the adsorbent utilizing an antigen-antibody reaction, followed by elution according to a known method such as a change in pH (lowering pH to 2.5 to 3, raising pH to 11.5 or higher, etc.), change in ionic strength (1 M NaCl, etc.), change in polarity (10% dioxane, 50% ethylene glycol, 3 M chaotropic salt ($SCN^-$, $CCl3COO^-$, $I^-$, etc.), addition of a protein denaturing agent (8 M urea, 6 M guanidine hydrochloride, etc.), dissociation by electrophoresis, and the like.

Then, endocrine disruptors, their degradation products and a mixture thereof which are present in trace amounts in an environment can be concentrated at a much higher concentration compared with conventional concentration methods such as solvent extraction, solid phase extraction and the like. Further, a concentrate with less contaminants which interfere with a determination can be obtained.

The following Examples further illustrate the present invention in detail but are not to be construed to limit the scope thereof.

Although the following Examples are directed to alkylphenol ethoxylates (APE), alkylphenols (AP), plastic resin components (PRC), plastic plasticizers (PP), and chlorophenols (CP) which have been known to be endocrine disruptors, they are applicable to other endocrine disrupting chemicals in the same way.

The hybridoma in the each Example has been deposited at National Institute of Bioscience and Human-Technology (NIBH), Agency of Industrial Science & Technology, Ministry of International Trade & Industry of 1-1-3 Higashi, Tsukuba-shi, Ibaraki-ken, Japan under Budapest Treaty. The accession number of each hybridoma and the date of deposition are listed in the following Table.

| Monoclonal antibody | Hybridoma | Accession number | Date of deposition |
|---|---|---|---|
| Anti-APE antibody | MOF3-139 | FERM BP-6059 | Aug. 13/1997 |
| Anti-AP antibody | AP-14 | FERM BP-6633 | Feb. 2, 1999 |
| Anti-PRC antibody | BP2-177 | FERM BP-6634 | Feb. 2, 1999 |
| Anti-PP antibody | DF-34 | FERM BP-6635 | Feb. 2, 1999 |
| Anti-CP antibody | CP-8 | FERM BP-6636 | Feb. 2, 1999 |

Example 1

Production of hapten monoclonal antibody

(1) Preparation of hapten

1) Preparation of hapten for antibody against alkylphenol ethoxylates and hapten for antibody against alkylphenols

Polyethylene glycol mononononylphenyl ether (NP5EO, 5.0 g) was dissolved in toluene (60 ml) and reacted with succinic anhydride (918 mg) and p-toluenesulfonic acid (16 mg) at 80°C for 2 hours under a nitrogen atmosphere. The reaction mixture was washed with ether under alkaline conditions (pH 12), followed by extraction with chloroform under acidic conditions (pH 2). The extract was dehydrated and evaporated to dryness to obtain the desired material (2.1 g).

2) Preparation of hapten for antibody against plastic resin components

Bisphenol A (25 g) and glutaric anhydride (12.5 g) were reacted at 100°C for 18 hours under a nitrogen atmosphere. The reaction mixture was dissolved in ethyl acetate, followed by purification with a silica gel column and an octadecyl silica gel (ODS) column, and crystallization from hexane to obtain the desired material (2.1 g).

3) Preparation of hapten for antibody against plastic plasticizers

8-Bromooctanoic acid (10 g) was dissolved in tetrahydrofuran (300 ml) and to this solution was added diphenylaminomethane (20 ml). The mixture was reacted at room temperature overnight. On the next day, diphenylaminomethane (20 ml) was further added and the mixture was reacted at room temperature overnight again. The reaction mixture was concentrated under reduced pressure, dissolved in hexane-ethyl acetate (9 : 1) and then roughly purified with a silica gel column.

The roughly purified product (20 g) together with phthalic acid (2.42 g) and 1,8-diazabicycloundecene (DBU, 2.22 g) in benzene (60 ml) were heated under reflux overnight. On the next day, DBU (2.22 g) was added and the mixture was further heated under reflux for 6 hours. After cooling to room temperature, water and chloroform were added to the reaction mixture and it was washed with water. The chloroform layer was dehydrated and concentrated, dissolved in hexane-ethyl acetate (9 : 1) and rough purified by a silica gel column.

The roughly purified product (4.1 g) was dissolved in tetrahydrofuran (THF, 100 ml) and 10% Pd/C (water content: 50%, 0.4 g) was added thereto. After bubbling hydrogen (0.3 ml/min. for 5 hours), 10% Pd/C (1.2 g) was further added thereto. After further bubbling hydrogen (0.5 ml/min. for 2 hours), the catalyst was removed and the reaction mixture was concentrated under reduced pressure. The reaction mixture was dissolved in 75% methanol and purified with an ODS column to obtain the desired material (1.9 g).

4) Preparation of hapten for chlorophenols

5-Bromovaleric acid (50 g) and triphenylphosphine (72.5 g) in toluene (400 ml) were heated under reflux for overnight. The reaction mixture was filtered and solids were washed with toluene and dried under reduced pressure to obtain phosphonium salt (120 g). Separately, p-hydroxybenzaldehyde (25 g) was dissolved in acetic acid (125 ml) (35°C). The temperature was raised to 50°C and chlorine gas was bubbled into the solution for 2 hours and then the mixture was allowed to cool to room temperature (35°C) to obtain white crystals (19 g). The crystals (19 g) was mixed with acetic acid (36 g) and the mixture was heated to 80°C. Chlorine gas was bubbled into the mixture until thick slurry was obtained. After cooling to room temperature, deposited white crystals were recrystallized from chloroform to obtain 3,5-dichloro-4-hydroxybenzaldehyde (9.3 g). These crystals (9 g), the above-obtained phosphonium salt (31.3 g) and t-butanol (15.5 ml) were dissolved in toluene (210 ml) and the solution was heated to 50°C. At this time, t-BuOK (23. 7 g) was added to the solution. After reaction at 55°C for 5 hours, the resultant reaction mixture was further reacted at room temperature overnight. The reaction mixture was poured onto ice and the toluene layer was discarded. The residual solution was adjusted to pH 2. After extraction with ethyl acetate, the reaction mixture was dried under reduced pressure, dissolved in hexane-ethyl acetate (9 : 1), and purified with a silica gel column to obtain a purified material (5.3 g). This purified product (2.55 g). 10% Pd/C (water content: 50%, 0.36 g), water (30 ml) and methanol (60 ml) were mixed and hydrogen was bubbled into the mixture (0.2 L/min. for 20 minutes) After removing the catalyst, the reaction mixture was concentrated under reduced pressure and extracted with isopropyl ether (IPE). After concentration under reduced pressure, the extract was purified with a silica gel column and an ODS column. The purified product was recrystallized from ether/hexane to obtain the desired material (1.7 g).

(2) Preparation of immunogen

Each hapten (1 mol), water-soluble carbodiimide (0.14 mol), N-hydroxysuccinimide (0.14 mol) was reacted in dimethylsulfoxide (2 ml) at room temperature overnight to form an activated ester. Then, bovine serum albumin (BSA, 10 mg) was dissolved in 0.13 M sodium bicarbonate ($NaHCO_3$) solution. The above activated ester (200 μl) was added thereto and reacted at 4°C overnight. Unreacted reagents were removed by ultrafiltration and the resultant was frozen and stored as the immunogen.

(3) Immunization

The complex of each hapten and BSA was dissolved in physiological saline at a concentration of 500 μg/ml and the solution was added to the equal amount of Freund's adjuvant or RIBI adjuvant. After thoroughly emulsifying, the emulsion was inoculated into BALB/c mice (female, 100 μg/mouse) subcutaneously and booster immunization was conducted 2 week intervals (Freund's) or 3 week intervals (RIBI). After immunizing booster 5 to 6 times, as the last immunization, the same complex solution (5 to 10 μg/0.1 ml physiological saline/mouse) was administered to an individual which showed the maximum serum antibody titer intravenously.

(4) Cell fusion

Three days after the last immunization, the spleen was excised from the abdomen of each mouse and a suspension of spleen cells (about $10^3$ cells) was prepared according to a conventional manner. Then, mouse myeloma cells (R3U1) (2 x $10^7$ cells) which had been washed with a serum-free culture medium three times were added thereto and subjected to cell fusion with PEG 6000 according to the method disclosed by Koehler and Milstein [Nature, 256, 495 (1975)].

After completion of the fusion, the cell mixture was suspended in so-called HAT medium containing hypoxanthine, aminopterin and thymidine and cultured for 10 days. Cells originating from the spleen died out during the 10 day-culture period spontaneously and P3U1 strain which did not fused together with the spleen cells also died out due to HAT medium. The cells which survived in the medium were regarded as hybridomas and then culture was continued by replacing the medium with HT medium corresponding to aminopterin-free HAT medium.

(5) Primary selection and cloning of hybridoma

An antibody titer of the supernatant of hybridoma culture was measured by ELISA using a microplate in which a complex of the hapten and OVA (hereinafter abbreviated to hapten-OVA) prepared by the same manner as that described with respect to the above immunogen was adsorbed. From 10 days to 20 days after fusion, the appearance of hybridomas were observed and the appearance of an antibody which bound to the hapten-OVA was recognized. A hybridoma which produced the antibody showing especially high binding activity was subjected to cloning by limiting dilution analysis.

(6) Secondary selection of hybridoma

For each hybridoma obtained by the method of the above (5), a objective compound (alkylphenol ethoxylates, alkylphenols, plastic resin components, plastic plasticizers, chlorophenols, etc.) was added to the supernatant of hybridoma culture and the hybridoma was subjected to binding inhibitory examination wherein inhibition of binding to a SPC-OVA-adsorbing microplate was examined and a hybridoma whose binding was inhibited by only the objective compound was selected.

Thus, a hybridoma which produced a monoclonal antibody specifically binding to each subject compound was obtained.

(7) Production of monoclonal antibody

The cloned hybridoma was incubated in Daigo T medium (Nippon Seiyaku, Tokyo, Japan) containing 10% bovine fetal serum at 37°C under 5% $CO_2$ and the antibody was obtained from the culture supernatant.

On the other hand, for obtaining a large amount of the antibody, 0.5 ml of mineral oil was administered to a BALB/c mouse intraperitoneally and then 5 x $10^6$ cells of the hybridoma were inoculated in the mouse intraperitoneally. After about 10 days, the collection of ascites fluid was observed in the mouse that received inoculation of the cells and the ascites fluid was harvested from the peritoneal cavity by abdominal section of the mouse.

The purification of the antibody was carried out by fractionating a fraction from the supernatant of cell culture

or ascites fluid with 45 to 50% saturated ammonium sulfate according to a conventional method and subjecting to column chromatography on a protein A column.

Example 2

Determination of alkylphenol ethoxylates (APE) and plastic resin components (PRC)

(1) Preparation of antigen-enzyme complex

According to a conventional manner, an activated ester of APE and a PRC hapten were bound to horseradish peroxidase, followed by removal of unreacted reactants by ultrafiltration to prepare each antigen-enzyme complex.

(2) Calibration curve

After mixing a standard solution and a solution of an antigen-enzyme complex, the mixture was added to a plate having a solid phase of each antibody. After reaction for 60 to 90 minutes, a color-producing substrate solution was added and the absorbance was measured. The calibration curves obtained by using nonylphenol ethoxylate (NPE) and bisphenol A (BPA) as the standard materials are shown in Fig. 2 and Pig. 3, respectively. The measurable ranges were 50 to 2000 µg/L of alkylphenol ethoxylates and 50 to 500 µg/L of plastic resin components.

Example 3

Determination of alkylphenols (AP), chlorophenols (CP) and plastic plasticizers (PP) by antigen solid phase method

The following example illustrates the determination by using anti-AP, CP, and PP monoclonal antibodies according to an antigen solid phase method.

(1) Calibration curve

After mixing a standard solution and a solution of each antibody, the mixture was added to a hapten-OVA solid phase plate. After reaction at 37°C for 60 minutes, an enzyme-labeled secondary antibody was added and the mixture was reacted at 37°C for 60 minutes. A color-producing substrate was added and the absorbance was measured.

The measurable ranges were 0.5 to 30 mg/L of alkylphenols (standard material: nonylphenol, Fig. 4), 30 to 500 mg/L of chlorophenols (standard material: 2,4,6-trichlorophenol, Fig. 5) and 0.1 to 30 mg/L of plastic plasticizers (standard material: dibutylphthalate, Fig. 6).

A hybridoma obtainable by fusing spleen cells or lymphocytes of an animal immunized by a complex of an endocrine disruptor or a compound similar thereto and a protein with myeloma cells, said hybridoma producing a monoclonal antibody against the endocrine disruptor or its degradation product.

The above hybridoma , wherein the endocrine disruptor is selected from the group consisting of alkylphenol ethoxylates, alkylphenols, plastic resin components, plastic plasticizers, and chlorophenols.

The above hybridoma , wherein the endocrine disruptor is selected from alkylphenols of the formula (1):

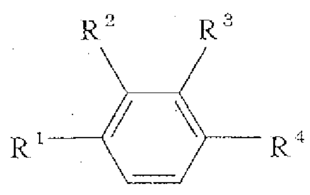

(1)

wherein $R^1$, $R^2$ and $R^3$ are the same or different, and each is H or straight or branched alkyl having 1 to 20 carbon atoms; R4 is $(OC_2H_4)_mOH$ or $(OC_2H_4)_mCOOH$; and m is an average number of ethylene oxide chains of 1 to 70.

The above hybridoma , wherein the endocrine disruptor is selected from alkylphenols of the formula (2):

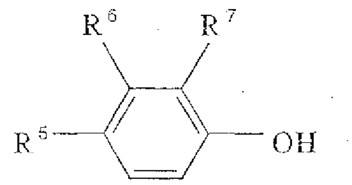

(2)

wherein $R^5$, $R^6$ and $R^7$ are the same or different, and each is H or straight or branched alkyl having 1 to 20 carbon atoms.

The above hybridoma , wherein the endocrine disruptor is selected from plastic resin components of the formula (3):

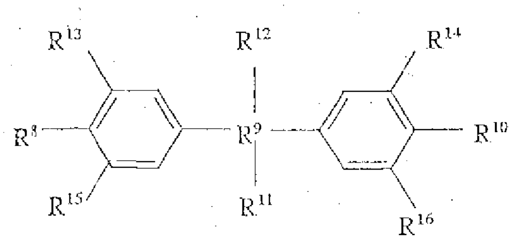

(3)

wherein $R^9$ is C, CO or $SO_2$; $R^8$ and $R^{10}$ are the same or different, and each is H, OH, $NH_2$ or $O(CH_2)_2OH$; $R^{11}$ and $R^{12}$ are the same or different, and each is absent, H, $CH_3$, $CH_2OH$ or $C_2H_4COOH$; and $R^{13}$, $R^{14}$, $R^{15}$ and $R^{16}$ are the same or different, and each is H, OH, $CH_3$, Cl or Br

The above hybridoma , wherein the endocrine disruptor is selected from plastic plasticizers of the formula (4):

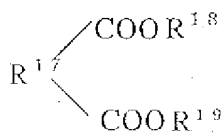

(4)

wherein $R^{17}$ is o-phenylene or tetramethylene; and $R^{18}$ and $R^{19}$ are the same or different, and each is H, straight or branched alkyl having 1 to 20 carbon atoms, benzyl or cyclohexyl.

The above hybridoma , wherein the endocrine disruptor is selected from chlorophenols of the formula (5):

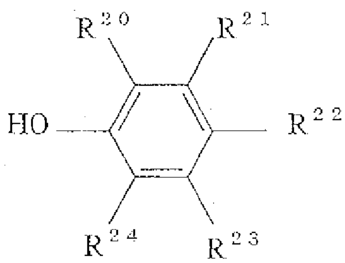

(5)

wherein $R^{20}$, $R^{21}$, $R^{22}$, $R^{23}$ and $R^{24}$ are the same or different, and each is H or Cl.

The above hybridoma which is mouse hybridoma MOF3-139, AP-14, BP2-177, DF-34 or CP-8

A monoclonal antibody against an endocrine disruptor or its degradation product which is produced by a hybridoma obtainable by fusing spleen cells or lymphocytes of an animal immunized by a complex of the endocrine disruptor or a compound similar thereto and protein with myeloma cells, said hybridoma producing a monoclonal antibody against the endocrine disruptor or its degradation product.

The above monoclonal antibody , wherein the endocrine disruptor is selected from the group consisting of alkylphenol ethoxylates, alkylphenols, plastic resin components, plastic plasticizers, and chlorophenols.

The above monoclonal antibody , wherein the endocrine disruptor is selected from the alkylphenol ethoxylates of the formula (1).

The above monoclonal antibody 10, wherein the endocrine disruptor is selected from the alkylphenols of the formula (2).

The above monoclonal antibody , wherein the endocrine disruptor is selected from plastic resin components of the formula (3).

The above monoclonal antibody , wherein the endocrine disruptor is selected from plastic plasticizers of the formula (4).

The above monoclonal antibody , wherein the endocrine disruptor is selected from chlorophenols of the formula (5).

The above monoclonal antibody which is produced by is mouse hybridoma MOF3-139, AP-14, BP2-177, DF-34 or CP-8.

A kit for immunological analysis of an endocrine disruptor, its degradation product or a mixture thereof comprising as an essential constitutional component the above monoclonal antibody.

A method for immunological analysis of an endocrine disruptor, its degradation product or a mixture thereof in a specimen which comprises reacting the specimen with the above monoclonal antibody of supported on a carrier, and the endocrine disruptor, its degradation product or the mixture thereof labeled with a labeling agent, and then determining an activity of the labeling agent either supported on the carrier or not supported on the carrier.

A kit for immunological concentration of an endocrine disruptor, its degradation product or a mixture thereof comprising as an essential constitutional component the above monoclonal antibody.

A method for immunological concentration of an endocrine disruptor, its degradation product or a mixture thereof in a specimen which comprises binding the endocrine disruptor, its degradation product or a mixture thereof in the specimen to the above monoclonal antibody supported on a carrier and then eluting the bound endocrine disruptor, its degradation product or the mixture to concentrate it.

## Claims

1. A monoclonal antibody against an endocrine disruptor being a plastic plasticizer or its degradation product which is produced by a hybridoma obtainable by fusing spleen cells or lymphocytes of an animal immunized by a complex of the endocrine disruptor or a compound similar thereto and protein with myeloma cells, said hybridoma producing a monoclonal antibody against the endocrine disruptor or its degradation product.

2. The monoclonal antibody according to claim 2, wherein the plastic plasticizer is selected from plastic plasticizers of the formula (4):

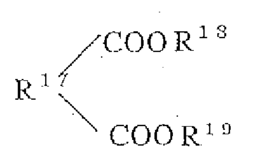

(4)

   wherein $R^{17}$ is o-phenylene or tetramethylene; and $R^{18}$ and $R^{19}$ are the same or different, and each is H, straight or branched alkyl having 1 to 20 carbon atoms, benzyl or cyclohexyl.

3. The monoclonal antibody according to claim 2 which is produced by mouse hybridoma DF-34 (FERM BP-6635).

4. A hybridoma as defined in claims 1 or 3 being capable of producing a monoclonal antibody as defined in claims 1 to 2.

5. The hybridoma of claim 4 which is mouse hybridoma DF-34 (FERM BP-6635).

6. A kit for immunological analysis of an endocrine disruptor, its degradation product or a mixture thereof comprising as an essential constitutional component the monoclonal antibody according to any one of claims 1 to 3.

**7.** A method for immunological analysis of an endocrine disruptor, its degradation product or a mixture thereof in a specimen which comprises reacting the specimen with the monoclonal antibody according to any one of claims 1 to 3 supported on a carrier, and the endocrine disruptor, its degradation product or the mixture thereof labeled with a labeling agent, and then determining an activity of the labeling agent either supported on the carrier or not supported on the carrier.

**8.** A kit for immunological concentration of an endocrine disruptor, its degradation product or a mixture thereof comprising as an essential constitutional component the monoclonal antibody according to any one of claims 1 to 3.

**9.** A method for immunological concentration of an endocrine disruptor, its degradation product or a mixture thereof in a specimen which comprises binding the endocrine disruptor, its degradation product or mixture thereof in the specimen to the monoclonal antibody according to any one of claims 1 to 3 supported on a carrier and then eluting the bound endocrine disruptor, its degradation product or the mixture to concentrate it.

Fig. 1

Chemical structure of hapten for each compound

1. For APE, AP antibodies 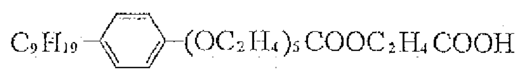

2. For PRC antibody 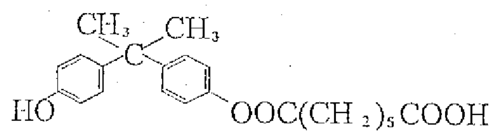

3. For PP antibody 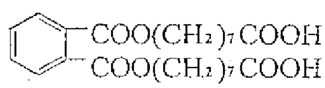

4. For CP antibody 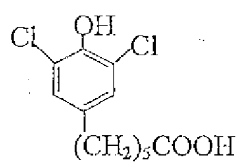

Fig. 2

Determination of alkylphenol ethoxylates
(antibody solid phase method)

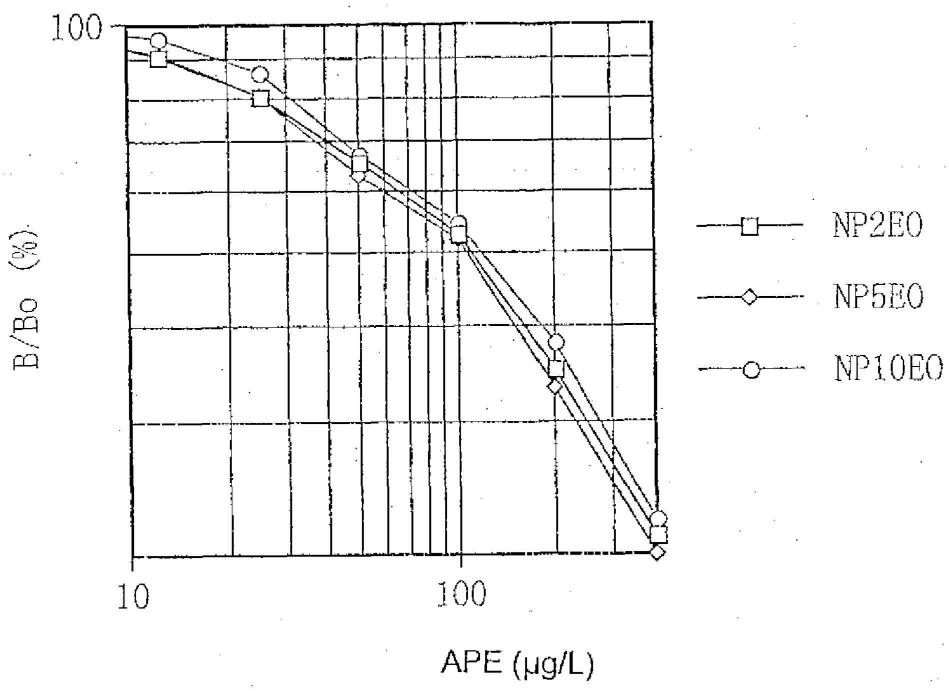

Fig. 3

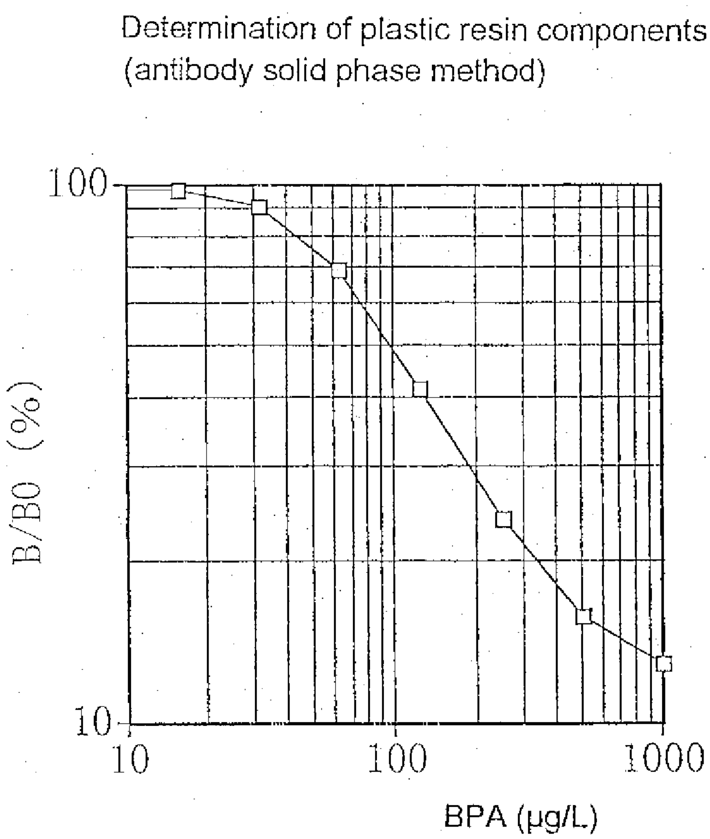
Determination of plastic resin components
(antibody solid phase method)
100
10
B/B0 (%)
10
100
1000
BPA (μg/L)

Fig. 4

Determination of alkylphenols
(antibody solid phase method)

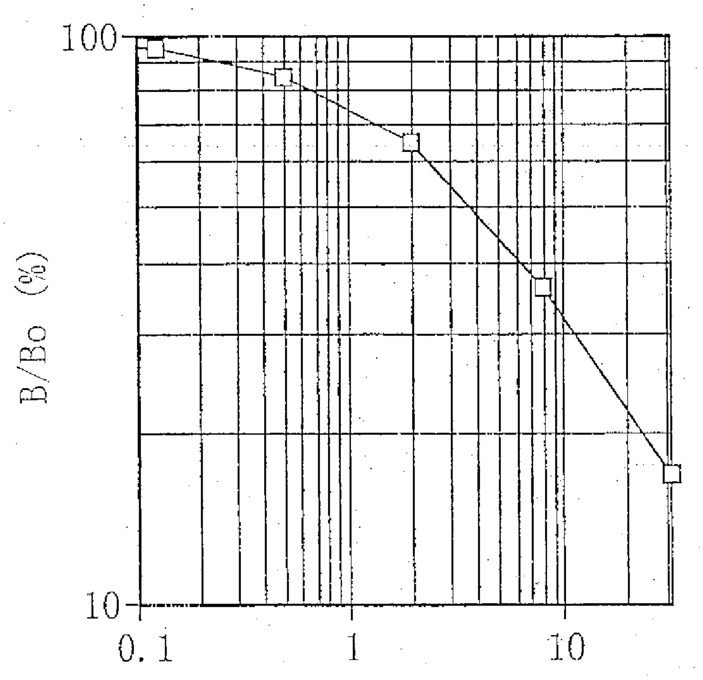

Fig. 5

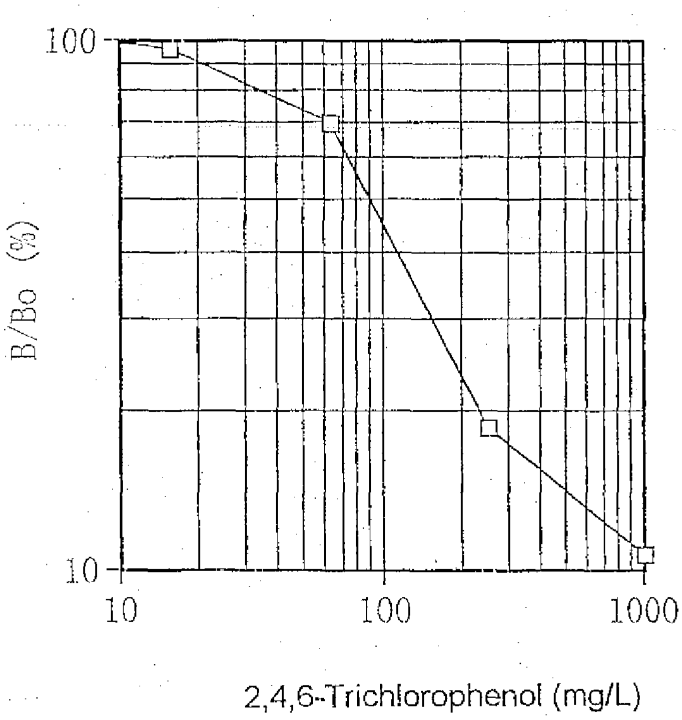

Fig. 6

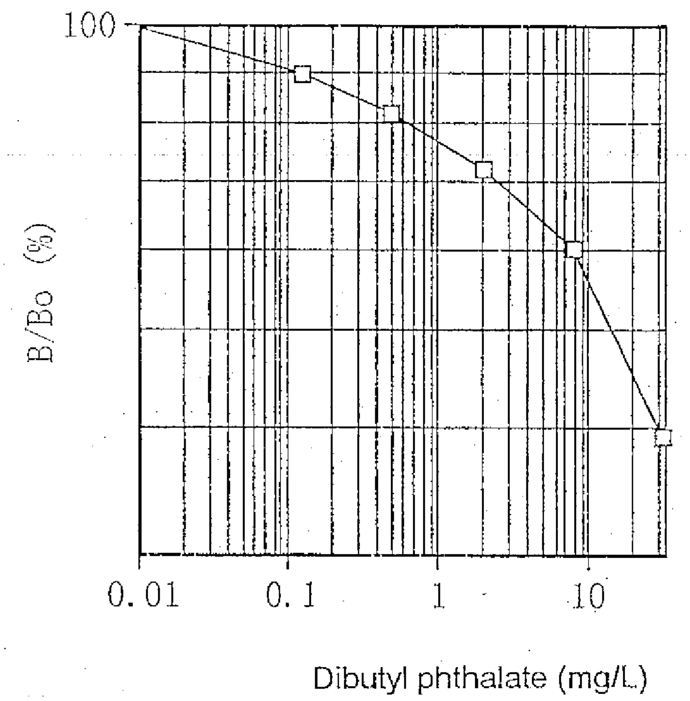

European Patent Office

# EUROPEAN SEARCH REPORT

Application Number
EP 05 10 5334

| Category | Citation of document with indication, where appropriate, of relevant passages | Relevant to claim | CLASSIFICATION OF THE APPLICATION (Int.Cl.6) |
|---|---|---|---|
| X | EP 0 242 589 A (RESEARCH CORPORATION) 28 October 1987 (1987-10-28)<br>* page 2, line 4 - line 22 *<br>* page 7, line 27 - line 29 *<br>* claims; examples * | 1-9 | C12N15/06<br>C12N5/20<br>C07K16/44 |
| Y | GASCON J ET AL: "Detection of endocrine-disrupting pesticides by enzyme-linked immunosorbent assay (ELISA): application to atrazine"<br>TRAC, TRENDS IN ANALYTICAL CHEMISTRY, ANALYTICAL CHEMISTRY. CAMBRIDGE, GB, vol. 16, no. 10, 12 November 1997 (1997-11-12), pages 554-562, XP004100825<br>ISSN: 0165-9936<br>* page 554 - page 562 * | 1-9 | |
| Y | CASTILLO M ET AL: "Analysis of industrial effluents to determine endocrine-disrupting chemicals"<br>TRAC, TRENDS IN ANALYTICAL CHEMISTRY, ANALYTICAL CHEMISTRY. CAMBRIDGE, GB, vol. 16, no. 10, 12 November 1997 (1997-11-12), pages 574-583, XP004100827<br>ISSN: 0165-9936<br>* page 574 - page 583 * | 1-9 | TECHNICAL FIELDS SEARCHED (Int.Cl.6)<br>C07K |

The present search report has been drawn up for all claims

| Place of search | Date of completion of the search | Examiner |
|---|---|---|
| Munich | 27 July 2005 | Rankin, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

## ANNEX TO THE EUROPEAN SEARCH REPORT ON EUROPEAN PATENT APPLICATION NO. EP 05 10 5334

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2005

| Patent document cited in search report | Publication date | Patent family member(s) | Publication date |
|---|---|---|---|
| EP 0242589 A | 28-10-1987 | AU 594134 B2 | 01-03-1990 |
| | | AU 7013787 A | 24-09-1987 |
| | | DK 140387 A | 19-09-1987 |
| | | EP 0242589 A2 | 28-10-1987 |
| | | FI 871174 A | 19-09-1987 |
| | | IL 81941 A | 21-11-1991 |
| | | JP 62272972 A | 27-11-1987 |
| | | NO 871115 A | 21-09-1987 |
| | | NZ 219676 A | 26-09-1990 |
| | | PT 84504 A ,B | 01-04-1987 |
| | | ZA 8701979 A | 25-11-1987 |

EPO FORM P0459

For more details about this annex : see Official Journal of the European Patent Office, No. 12/82